# EUROPEAN PATENT SPECIFICATION

(11) **EP 1 360 958 B1**
(45) Date of publication and mention of the grant of the patent: **13.09.2006**
(21) Application number: 01953764.6
(22) Date of filing: 17.07.2001
(51) Int. Cl.: A61K 9/127

(54) **LIPOSOMIC FORMULATION OF CLOBETASOL PROPIONATE**
LIPOSOMENFORMULIERUNG MIT CLOBETASOL PROPIONATE
FORMULATION LIPOSOMIQUE DE PROPIONATE DE CLOBETASOL

(30) Priority: 17.07.2000 CU 1712000
(43) Date of publication of application: 12.11.2003
(73) Proprietor: Centro de Investigation y Desarrollo de Medicamentos (CIDEM), Ciudad Habana 10600 (CU)
(72) Inventor: PEREZ GUTIERREZ, Xiomara, Boyeros, Ciudad Habana 10800 (CU); GARCIA LEON, Ladyth de la Caridad, Ciudad Habana 10300 (CU); DE LA PAZ MARTIN-VIANA, Nilia, Guanabacoa, Ciudad Habana 10400 (CU); ROCHE GONZALEZ, Aymeé, Ciudad Habana 12600 (CU); HERNANDEZ GONZALEZ, Ignacio, Ciudad Habana 12600 (CU); ALBERTOVNA DIDUK, Natalia, Ciudad Habana 11300 (CU); GRANDA CORTADA, D., Calle Linea 61 entre M y N, Ciudad Habana 10400 (CU)
(74) Representative: Prins, Adrianus Willem
(86) International application number: PCT/CU2001/000005
(87) International publication number: WO 2002/007702

(56) References cited:
- US-A- 4 427 649
- US-A- 5 192 528
- JACOBS A ET AL: "Effects of Phosphatidylcholine on the Topical Bioavailability of Corticosteroids Assessed by the Human Skin Blanching Assay" J PHARM PHARMACOL, vol. 40, 24 Junio 1988 (1988-06-24), páginas 829-833, XP002902000

## Description

The present invention relates to a dermatological composition comprising liposomes with Clobetasol Propionate and is within the field of liposomal products used in human medicine.

As is already known, Clobetasol Propionate is a powerful synthetic corticosteroid with anti-allergic, anti-exudative and anti-proliferative properties, which inhibit the inflammatory response produced by mechanical, chemical or immunological agents. It is prescribed in cream-form (CLOVATE® ) or as an ointment (DECLOBAN® ) for the short term treatment of dermatoses such as psoriasis, recidivist eczemas, flat lichen discoid lupus erythematosus, which are resistant to the commonly used external corticosteroids. Nevertheless, the use of this medicine has been restricted as maximum up to 14 days due to the side effects produced, particularly the hypophysis-adrenal inhibition, risk that increases with the preparation potency, as well as with the application surface and with the duration of the therapy.

The patent applications WO 93/15740, EP 0391033, WO 98/36753, WO 96/27376, JP11130679, as well as the patent US 5998395 relate to formulations comprising steroid anti-inflammatory drugs including Valerate and Clobetasol Propionate. In these compositions the Clobetasol Propionate is dispersed and although they head to increase the therapeutic activity of the preparation, they are not able to diminish the adverse effects associated to the use of the medication.

On the other hand, the patents US 5192528, US 4427649, WO 97/13500, US 4693999, WO 96/22764 and US 5190936 refer to liposomal formulations of corticosteroids but none comprise the encapsulation of the Clobetasol Propionate or any other derivative of the aforementioned corticosteroid in liposomes.

### Disclosure of the Invention.

The present invention is related with a liposomal formulation of Clobetasol Propionate for topical use, which is characterized because it uses low concentrations of the active ingredient (between 0,01 and 0,03) and it renders high concentrations of the product in the skin, being accentuated more this effect in the stratum corneum, from where the product can be control released and it diminishes the diffusion of the active ingredient toward the systemic circulation, minimizing this way the toxicity of the same, and therefore its adverse effects.

The efficiency of the composition of the invention is similar to the classical cream and ointment formulations where the Clobetasol Propionate is diffused, although surprisingly it was observed that encapsulating said active ingredient in liposomas, its concentration can be reduced a half of the amount used in classic formulations of this active ingredient maintaining its powerful anti-inflammatory effect, due to the high affinity of the compounds belonging to the lipid double-layer of liposomes with the biological membranes, achieving a maxim local action.

The current invention provides a liposomal formulation for external use, preferably in gel form, comprising Clobetasol Propionate to low concentrations, phospholipids, a lipophilic additive, antioxidant agents and a solvent for Clobetasol Propionate, like phospholipids, lipophilic additives and antioxidant agents, and also aqueous solution forming part of the liposomal suspension, as well as conventional auxiliary agents in geles or the appropriate external formulations: creams, ointments and lotions.

The present composition preferably includes a liposomal suspension, of multi-lamellar vesicles with sizes varying between 2000 and 5000 nm, preferably between 3500 and 4500 nm.

The compositions of the invention, comprise, in weight of the total weight of the composition the following essential components:
- Phospholipids suitable for use in the formulation are, for example, phosphatidyl cholines, phosphatidylethanolamines and phosphatidylserines, and mixtures thereof, in particular those soluble along with Clobetasol Propionate in a non-toxic, organic and pharmaceutically acceptable solvent. Among them are preferred the phosphatidylcholine or lecithin, soyabean-lecithin for example. The amount of phospholipid in the present formulation may vary between 1.0 and 1.5 %, and is preferably 1.2 %.
- Lipophilic additives to selectively modify the characteristics of the liposomes. These may include cholesterol, phosphatidic acid, estearylamine, cholesterol being the ideal additive. The amount of lipophilic additive may vary between 0.1 and 0.6 %, preferably between 0.2 and 0.4 % and is preferably 0.3 %. In general the ratio of lipophilic additive to phospholipid varies between 1:1 and 1:3, and in this case was 1:2.5.
- Anti-oxidant agents for liposome preparation, among them α-tocopherol and butylated hydroxytoluene (BHT) may be used as anti-oxidant agents. BHT is the ideal.
   For the formulation preparation, the above mentioned phospholipids, additives, anti-oxidants and active ingredient (Clobetasol Propionate) are dissolved in a watersoluble, non-toxic; and pharmaceutically acceptable organic solvent.
- The organic solvent used in the liposomal suspensions may be a glycol, particularly propylene glycol, in quantities ranging between 8 and 15 %, particularly between 10.0 and 12.5 % and preferably 11.8 %.
   The term "organic component" used here refers to mixtures composed of the phospholipid, lipophilic additive, anti-oxidant and the organic solvent.
- The active ingredient, Clobetasol Propionate is dissolved in the organic component. The quantity of the active ingredient in the final composition varies between 0.01-0.06 %, preferably between 0.02 - 0.055 % and preferably between 0.02 - 0.05 %.
- The liquid component of the present formulation is formed principally of water and may contain various additives such as electrolytes or preferably a buffer system, preferably the Tris HCI buffer. Such a buffer system will maintain the pH of the liquid component at an interval of between 5 and 7.5, preferably between 6 and 7.2 and preferably between 6.5 and 7.
   The present invention includes the finished gel product for external use containing preservatives, anti-oxidants and the normal ingredients used in gel manufacture.
- Among the preservatives that may be used to avoid degradation by microorganisms are benzoic acid, methylparaben and propylparaben. The two latter preservatives are preferably used in quantities of 0.2 % and 0.02 % respectively.
- Among the anti-oxidants that may be used for preserving the composition are tetracetic ethylenediamine acid (EDTA), disodic edetate and sodium methabisulphite. The optimum being either EDTA or sodium methabisulphite at 0.01 % and 0.2 % respectively.
- The composition of the invention also includes suitable gelling agents for obtained the end form for external use, these agents may for example hydroxypropylmethylcellulose, hydroxyropylcellulose, their mixtures or carbopol 940, being preferred carbopol 940, together with triethanolamine. The amount of these gelling agents will depend of the remainder ingredients and in general is approximately from 0.5 to 1.0%, and in particular is about 0.85% for carbopol 940 and 0.65% for triethalonamine.

For preventing degradation of the materials used during the preparation of the liposomal formulation, it is advantageous to purge all solutions with an inert gas like argon or nitrogen, and to carry out all the steps under inert atmosphere.

The ideal proportions in the finished product, based on the total weight of the composition, are:
(a) 0.01 to 0.03 % of Clobetasol Propionate;
(b) 0.5 to 4.0 % of phospholipid;
(c) 0.1 to 0.6 % of cholesterol;
(d) 8 to 15 % of propylene glycol;
(e) 0.003 to 0.007 % of BHT;
(f) buffer agent to maintain the pH of the liquid component at an interval of 5 to 7.5;
(g) sufficient dermatologically acceptable preservatives and anti-oxidants to prevent degradation;
(h) a basic agent to regulate the pH of the gel;
(i) 0.5 to 1.0 % of gelling agents;
(j) water .

The preferred compositions would be those in which:
the quantity of Clobetasol Propionate is 0.02 to 0.025 %
the quantity of phospholipid is 1.0 to 1.5 %
the quantity of cholesterol is 0.2 to 0.4 %
the quantity of propylene glycol is 10 to 12.5 %
the quantity of gelling agent is 0.6 to 0.9 %

The optimum composition includes approximately (where weights are based on the total weight of the composition):
(a) 0.025 % of Clobetasol Propionate
(b) 1.2 % of phospholipid
(c) 0.3 % of cholesterol
(d) 11.8 % of propylene glycol
(e) 0.005 % de BHT
(f) 0.1 % of Tris (hydroxymethyl) - aminomethane and 0.02 % of hydrochlorhydric acid
(g) 0.2% of methylparaben and 0.02 % of propylparaben
(h) 0.01 % of EDTA and 0.2 % of sodium methabisulphite
(i) 0.65 % of triethanolamine
(j) 0.85 % of Carbopol 940
(k) water
The liposomal formulation of Clobetasol Propionate in the present invention is preferably prepared in the following way:
(a) heating the lipophilic additive and a portion of the organic solvent to between 100 and 140°C, preferably between 120 and 135°C in an initial beaker leaving the mixture to cool to between 70 and 90°C, dissolving in this the phospholipid, the active ingredient and the anti-oxidant and stirring until complete dissolving has occurred;
(b) heating in a second beaker the buffer solution, preferably at between 70 and 90°C, and adding part of this to the first beaker whose temperature should be maintained at between the 70 to 90°C mark until a paste is obtained;
(c) transferring the remainder of the second glass, still at between 70 and 90°C, to the first glass and mixing the combination in a high efficiency mixer;
(d) dissolving the preservatives in the organic solvent in a third beaker, preferably in the mixer, until complete dissolution;
(e) adding to a portion of the water the contents of the third beaker, preferably in a mixer, dissolving the anti-oxidants and the gelling agent, adding to the rest of the water dissolved triethanolamine and the contents of the first beaker, preferably maintaining the mixing action to homogenise.

At stage (c) any type of mixing machine or homogenizer which employs an intense cutting action on the mixture may be used. Generally, a mixer capable of reaching speeds of 2000 to 16000 rpm, particularly between 2500 and 6000 r.p.m. is ideal. In the following stages any mixing method may be used, particularly those which use a propeller-type mixing rod.

The liposomes prepared in the described manner, generally contain the highest amount of encapsulated active ingredient and the separation of the non-encapsulated liposomes is not necessary.

### Examples.

### Example 1. Preparation of the formulation of encapsulated Clobetasol Propionate.

The following proportions of components in the formulation were used (Table 1).

| Ingredient | Quantity, g/100g of gel |
|---|---|
| Clobetasol Propionate | 0.025 |
| Hydrogenated soyabean- lecithin | 1.200 |
| Cholesterol | 0.300 |
| Propylene glycol | 11.800 |
| Butylated hydroxytoluene | 0.005 |
| Tris | 0.100 |
| Hydrochlorhydric acid | 0.020 |
| Methylparabene | 0.200 |
| Propylparabene | 0.020 |
| Enthylenediaminotetracetic acid | 0.010 |
| Sodium methabisulphite | 0.200 |
| Carbopol 940 | 0.850 |
| Triethanolamine | 0.650 |
| Water | 84.595 |

### Procedure

1. The cholesterol is heated in approximately a 6th part of the propylene glycol to 120 - 135°C. The mixture is left to cool to approximately 70 - 90°C. The Clobetasol Propionate, soyabean-lecithin and the butylated hydroxytoluene are dissolved in this mixture at 70 - 90°C. Complete dissolving is achieved through stirring.
2. 0.1g of Tris (hydroxymethyl)-aminomethane and 0.02 g of hydrochlorhydric acid are dissolved in approximately a quarter part of the water in a separate beaker which is kept at 70 - 90°C. Approximately 5 ml of this solution is extracted and added to the mixture obtained in step 1. This is mixed until a paste is formed.
3. The remainder of step two's mixture is added to the paste at between 70 and 90°C. This is homogenized for five minutes at 3000 r.p.m. in a high efficiency mixer.
4. The parabens are dissolved in the remaining propylene glycol in a separate beaker and complete dissolution is obtained through constant stirring.
5. Approximately half of the water is added to the result of stage 4 and stirred constantly. The EDTA and sodium methabisulphite are added and constantly stirred until completely dissolved. The carbopol 940 is gradually added and stirred vigorously and constantly for 30 minutes. The dissolved triethanolamine is added to the rest of the water and the result of stage 3 is immediately incorporated.
The homogenizer used was the POLYTRON® disperser with its efficient cutting action and the mixer used in stages 4 and 5 was a mechanical stirrer IKA which has a propeller rod.

The liposomal gel obtained from stage 5 can be apportioned into appropriate containers such as aluminium tubes coated in PVC.

### Example 2. Trial formulation in edema in ear of mice induced by croton oil.

Groups of ten animals were made up (male mice weighing from 28 to 32 g) and the edema was induced in the right ear through the application of 50 µl of croton oil (5 %) and ethanol (95 %) (v/v). The same volume of ethanol was applied in the left ear. Topical administration of the trial formulations was applied 30 minutes before the induction of the edema. In every case, 20 mg of the sample was applied to each ear. The negative control group only received the croton oil.

The following experimental groups were formed:

| Groups | Dose (µg of clobetasol/ear) |
|---|---|
| DECLOBAN® ointment | 10 |
| Clovate® cream | 10 |
| Liposomal gel of Table 1 | 5 |
| Control | No treatment |

1,2,3,4,5 and 6 hours after the induction of the edema all the groups of animals were killed by cervical dislocation. Both ears were removed to obtain and weigh discs of 5 mm in diameter.

In Table 2 the average values of weight difference of the ear according to treatment groups are shown. It may be seen that all the experimental groups are statistically different (p<0.05) to the control group in the interval of 4 to 6 hours after the induction, which demonstrates the inhibiting effect of the ointment, cream and liposomal preparation in the trial doses.

**TABLE 2**

| Time (h) | Control X± DS | Ointment 10µg/ear | Cream 10µg /ear | Liposomal gel 5µg /ear |
|---|---|---|---|---|
| | | X± DS | X± DS | X± DS |
| 1 | 0.77±0.7 | 1.70±1.1 | 2.74±1.6 | 1.08±0.6 |
| 2 | 3.17±2.0 | 2.65±2.7 | 2.51 ±0.9 | 1.86±0.6 |
| 3 | 6.84±2.8 | 3.16±1.6 | 4.47±2.1 | 5.03±2.4 |
| 4 | 7.72±2.6 | 2.35±1.5 | 5.17±2.4 | 4.41 ±3.7 |
| 5 | 9.42±1.9 | 3.47±2.4 | 3.86±2.2 | 4.26±2.6 |
| 6 | 7.76±2.0 | 3.51±2.8 | 2.88±1.7 | 2.95±2.7 |

The statistical analysis demonstrated that there were no significant differences at any of the studied times between the ointment, the cream and the liposomal gel at a dose of 5 (0.025 %) µg of Clobetasol Propionate per ear. A greater inhibition of the edema was found in the group treated with the ointment at the interval of 4 and 5 hours respectively.

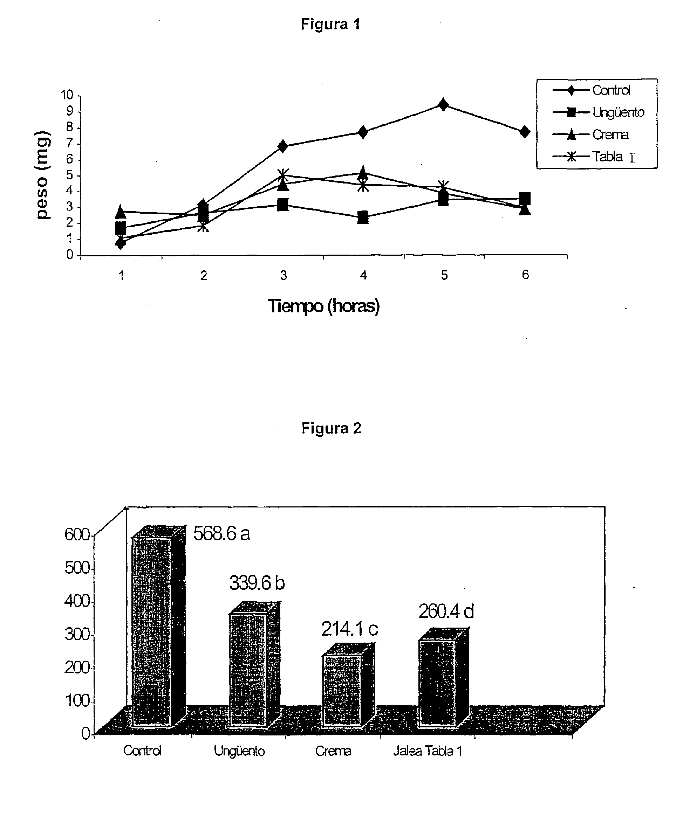

The inhibitory effect on the edema induced by the croton oil is shown in Figure 1.

The inhibitory response was similar for the groups treated with clobetasol propionate but it was higher for the groups treated with the cream and liposomal gel at a dose of 5 µg/ear.

Comparing the results obtained for different groups treated with liposomal gel, at maximum inflammation time (5 hours) the highest inhibition of the edema was observed.

### Example 3. Trial formulation in granuloma induced by cotton pellet.

Male Wistar rats with a body weight of between 250 and 300 g were used. These were kept in a temperature-controlled room of 2°C ± 20°C and a 12-hour light/darkness cycle.

The animals were anaesthetized intraperitoneally (IP) with 30 mg/kg of sodic phentobarbital. An incision was made in the dorsal area and two cotton pellets of 35 ± 2 mg were inserted, settling 2 cm from the wound. This was sewn and an antiseptic solution was applied to avoid sepsis. The animals were topical treated (0.3 g sample/cotton pellet) for 7 days and on the eighth they were killed in an ether atmosphere and the granulomas were extracted and weighed. The granulomas were dried in an oven at a constant 65°C over approximately 24 hours to determine the water content of the granuloma (wet weight - dry weight) and the fibre granularity (dry weight - cotton pellet weight). The following experimental groups were formed:

| Groups | Dose (µg of clobetasol/pellet) |
|---|---|
| DECLOBAN® ointment | 150 |
| Clovate® cream | 150 |
| Liposomal gel of table 1 | 75 |
| Control | No treatment |

An increased dry weight of the cotton pellet was observed in all treated groups during the assay.

The subcutaneous insertion of the cotton pellets provoked a vascular fibrous capsule with the presence of mononuclear cells; it resulted in a granuloma at the site of implantation.

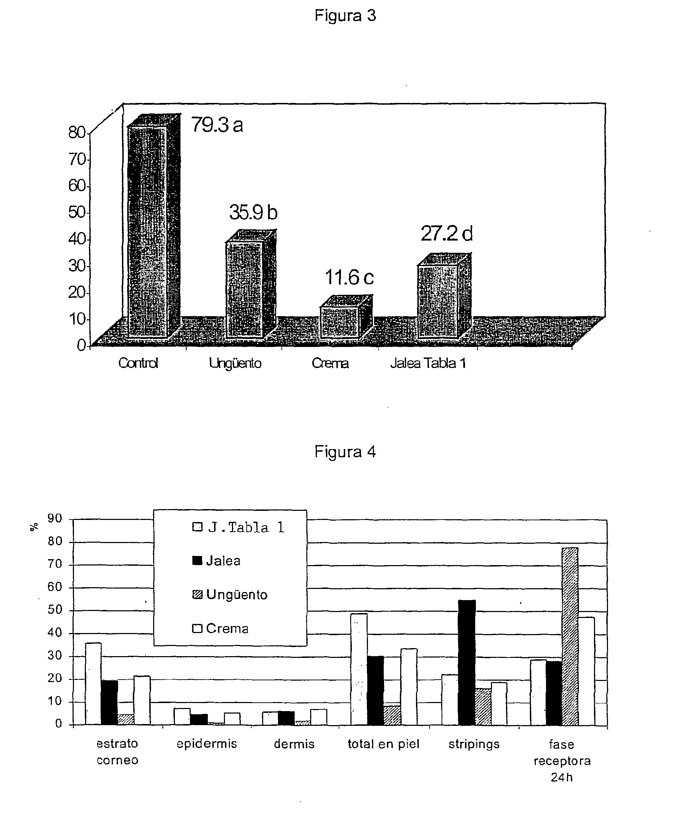

The water and fibre granularity content obtained after the application of the trial substances for seven days appear in figures 2 and 3.

Figure 2 shows the results obtained for different formulations containing Clobetasol Propionate. It was observed in all groups an inhibitory effect with the following order: Cream>liposomal gel of table 1> Ointment. It may be seen that all the experimental groups are statistically different (p<0.05) related to the aqueous content to the control group. From the results it can be concluded that the Ointment, the cream and the gel exert an inhibitory effect at the assayed doses.

Figure 3 shows the results obtained for different formulations containing Clobetasol Propionate. It was observed in all groups an inhibitory effect with the following order: Cream>liposomal gel of table 1> Ointment. It may be seen that all the experimental groups are statistically different (p<0.05) related to the fibre granularity content to the control group. From the results it can be concluded that the Ointment, the cream and the liposomal gel exert an inhibitory effect at the assayed dose.

The encapsulation of Clobetasol Propionate into liposomes is an advantage because the dose may be reduced to a half of the conventional dose used for other formulations and even the same therapeutic effect is obtained.

The results obtained in the pre-clinical study indicate that the liposomal gel of Clobetasol Propionate 0.025% have a topical anti-inflammatory effect in the experimental model evaluated, similar and in some cases superior to the effect observed in the conventional cream and ointment.

### Example 4. Study of "in vitro" percutaneous absorption.

Formerly the radiolabelling of the Clobetasol Propionate with tritium was carried out through an isotopic, catalytic, heterogeneous exchange in solid phase. A specific activity of 150 mCi/mmol at 95 % radiochemical purity was obtained. The labelled Clobetasol Propionate was mixed with the different subject formulations, these being:
Liposomal gel of Clobetasol Propionate at 0.025 %
Clobetasol Propionate gel at 0.05 %
Clobetasol Propionate cream at 0.05 %
Clobetasol Propionate ointment at 0.05 %

The human skin used in the "in vitro" percutaneous absorption study was obtained from aesthetic breast surgery.

Samples were taken from the receptor compartment 24 hours after the formulations were applied on the skin. Subsequently, the "stripping" process was carried out and the stratum corneum, epidermis and dermis layers were separated. Measurement of the radioactivity in each of the samples taken was through liquid scintillation. The stripping in this manner fundamentally represents the residue of the application, although the most superficial layers of the stratum corneum are also present.

The percentages of radioactivity accumulated in the samples, measured in relation to the activity applied in each case, are shown in Figure 4. It is demonstrated that the liposomal formulation deposit larger concentrations of the Clobetasol Propionate on the skin (approximately 50% of the applied dose), this being more pronounced on the stratum corneum with lower diffusion towards the receiving compartment as compared to the conventional formulations, even when the Clobetasol Propionate dose was reduced to a half.

On the other hand, it was observed that in the non-liposomal formulations the greatest quantity of Clobetasol Propionate was found in the receiving compartment, particularly with the ointment at 70 %. From these results one may expect a lower diffusion of the Clobetasol Propionate incorporated in liposomes towards the systemic circulation after "in vivo" application.

### Brief Description of the Figures

Figure 1: Shows the inhibitory effect of the formulation against the edema induced by the croton oil during the study period, through comparison of differences in ear weight (milligrams) against time in hours.
Figure 2: Shows the water content values obtained after 7 days of treatment with the various trial substances.
Figure 3: Shows the fibro-granularity content after 7 days of treatment with the various trial substances. In the figure, equal letters show no significant differences according to Duncan's test.
Figure 4: Shows the radioactivity count accumulated in the analyzed samples and for each of the evaluated formulations in relation to the radioactivity applied.

## Claims

1. A liposomal formulation for topical use, which contains;
(a) Clobetasol Propionate encapsulated in liposomes;
(b) a phospholipid;
(c) a lipophilic additive;
(d) anti-oxidant agents;
(e) an organic solvent;
(f) a buffer agent to maintain the pH level of the water component in the interval of 5 to 7.5;
(g) preservative substances;
(h) gelling agents;
(i) water

2. Formulation according to claim 1, wherein the liposomes are multi-lamellar vesicles.

3. Formulation according to claim 2, wherein the liposome sizes varying between 2000 and 5000 nm, preferably between 3500 and 4500 nm.

4. Formulation according to claim 1, wherein the phospholipid is selected from the group consisting in phosphatidylcholine, phosphatidylethanolamine and phosphatidylserine, Iysophosphatidylcholine and mixtures thereof,.

5. Formulation according to claim 4, wherein the phospholipid is lecithin, preferably soyabean- lecithin.

6. Formulation according to claim 5, wherein the lecithin is between 0.5 and 4.0 % (weight/weight), preferably between 1.0% and 1.5%, and in particular it is about 1.2%.

7. Formulation according to claim 1, wherein the lipophilic additive is selected from the group consisting in cholesterol, phosphatidic acid and estearylamine, being cholesterol the preferred additive.

8. Formulation according to claim 7, wherein the amount of lipophilic additive is between 0.1 and 0.6% (weight/weight), preferably between 0.2 and 0.4% and in particular it is approximately 0.3%.

9. Formulation according to claim 1 wherein the relationship between quantity of preservative lipophilic and the quantity of phospholipid varies between 1:1 and 1:3, and in particular it is approximately 1:2.5.

10. Formulation according to claim 1 wherein the anti-oxidant agent used in the preparation of the liposomas is α-tocopherol or butylated hydroxytoluene (BTH), being preferably BHT which is between 0.003 to 0.007% (weight/weight).

11. Formulation according to claim 1 wherein the anti-oxidant agent used for the preservation in the in the end form are ethylenediamine tetracetic acid (EDTA), disodic edetate and sodium methabisulphite being preferably either EDTA or sodium methabisulphite at 0.01 % and 0.2 % respectively.

12. Formulation according to claim 11 wherein the organic solvent is a glycol, and preferably it is propylene glycol.

13. Formulation according to claim 12 wherein the organic solvent is between 8 and 15% (weight/weight), in particular between 10.0 and 12.5% and preferably at 11.8%.

14. Formulation according to claim 1 wherein the buffer agent for maintaining the pH of the watery component between 5 at 7.5 is Tris (hydroxymethyl)-aminomethane at 0.1% (weight/weight) and hydrochlorhydric acid at 0.02% (weight/weight).

15. Formulation according to claim 1 wherein the preservative substances is methylparaben to 0.2% (weight/weight) and propylparaben at 0.02% (weight/weight).

16. Formulation according to claim 1 wherein the gelling agent is selected of the group consisting in hydroxypropylmethylcellulose, hydroxyropylcellulose, their mixtures or carbopol 940, being preferred carbopol 940 at 0.85%.

17. Formulation according to any of claims from 1 to 16 in end form of gel.

## Patentansprüche

1. Liposomale Formulierung zur topischen Anwendung, enthaltend:
(a) in Liposome eingeschlossenes Clobetasol-Propionat;
(b) ein Phospholipid;
(c) ein lipophiles Additiv;
(d) Antioxidationsmittel;
(e) ein organisches Lösungsmittel;
(f) ein Puffermittel, um den pH-Wert der wässrigen Komponente in dem Bereich von 5 bis 7,5 zu halten;
(g) Konservierungssubstanzen;
(h) Geliermittel;
(i) Wasser.

2. Formulierung nach Anspruch 1, worin die Liposome multi-lamellare Vesikel sind.

3. Formulierung nach Anspruch 2, worin die Liposomgrößen zwischen 2000 und 5000 nm variieren, bevorzugt zwischen 3500 und 4500 nm.

4. Formulierung nach Anspruch 1, worin das Phospholipid ausgewählt ist aus der Gruppe, die aus Phosphatidylcholin, Phosphatidylethanolamin und Phosphatidylserin, Lysophosphatidylcholin und Mischungen davon besteht.

5. Formulierung nach Anspruch 4, worin das Phospholipid Lecithin ist, bevorzugt Sojabohnen-Lecithin.

6. Formulierung nach Anspruch 5, worin das Lecithin zwischen 0,5 und 4,0 % (Gewicht/Gewicht) beträgt, bevorzugt zwischen 1,0 % und 1,5 %, und insbesondere etwa 1,2 % beträgt.

7. Formulierung nach Anspruch 1, worin das lipophile Additiv ausgewählt ist aus der Gruppe, die aus Cholesterin, Phosphatidsäure und Stearylamin besteht, wobei Cholesterin das bevorzugte Additiv ist.

8. Formulierung nach Anspruch 7, worin die Menge an lipophilem Additiv zwischen 0,1 und 0,6 % (Gewicht/Gewicht) beträgt, bevorzugt zwischen 0,2 und 0,4 % und insbesondere ungefähr 0,3 % beträgt.

9. Formulierung nach Anspruch 1, worin das Verhältnis zwischen der Menge an lipophilem Konservierungsmittel und der Menge an Phospholipid zwischen 1:1 und 1:3 variiert, und insbesondere ungefähr 1:2,5 beträgt.

10. Formulierung nach Anspruch 1, worin das bei der Herstellung der Liposome verwendete Antioxidationsmittel α-Tocopherol oder butyliertes Hydroxytoluol (BHT) ist, wobei BHT bevorzugt ist, das zwischen 0,003 bis 0,007 % (Gewicht/Gewicht) beträgt.

11. Formulierung nach Anspruch 1, worin das für die Konservierung in der Endform verwendeten Antioxidationsmittel Ethylendiamintetraessigsäure (EDTA), Dinatriumedetat und Natriummethabisulphit ist, wobei es bevorzugt entweder EDTA oder Natriummethabisulphit mit 0,01 % bzw. 0,2 % ist.

12. Formulierung nach Anspruch 11, worin das organische Lösungsmittel ein Glycol ist, und es bevorzugt Propylenglycol ist.

13. Formulierung nach Anspruch 12, worin das organische Lösungsmittel zwischen 8 und 15 % (Gewicht/Gewicht) beträgt, insbesondere zwischen 10,0 und 12,5 % und bevorzugt 11,8 %.

14. Formulierung nach Anspruch 1, worin das Puffermittel zum Aufrechterhalten des pH der wässrigen Komponente zwischen 5 und 7,5 Tris(hydroxymethyl)-Amionomethan mit 0,1 % (Gewicht/Gewicht) und Salzsäure mit 0,02 % (Gewicht/Gewicht) ist.

15. Formulierung nach Anspruch 1, worin die Konservierungssubstanzen Methylparaben mit 0,2 % (Gewicht/Gewicht) und Propylparaben mit 0,02 % (Gewicht/Gewicht) sind.

16. Formulierung nach Anspruch 1, worin das Geliermittel ausgewählt ist aus der Gruppe, die aus Hydroxypropylmethylcellulose, Hydroxypropylcellulose, ihren Mischungen oder Carbopol 940 besteht, wobei Carbopol 940 mit 0,85 % bevorzugt ist.

17. Formulierung nach einem der Ansprüche 1 bis 16 in Endform eines Gels.

## Revendications

1. Formulation liposomique pour utilisation topique, qui contient :
(a) du propionate de clobétasol encapsulé dans des liposomes ;
(b) un phospholipide ;
(c) un additif lipophile ;
(d) des agents antioxydants ;
(e) un solvant organique ;
(f) un agent tampon pour maintenir le niveau de pH du composant aqueux dans l'intervalle de 5 à 7,5 ;
(g) des substances conservatrices ;
(h) des agents gélifiants ;
(i) de l'eau.

2. Formulation selon la revendication 1, dans laquelle les liposomes sont des vésicules multilamellaires.

3. Formulation selon la revendication 2, dans laquelle la taille des liposomes varie de 2000 à 5000 nm, de préférence de 3500 à 4500 nm.

4. Formulation selon la revendication 1, dans laquelle le phospholipide est choisi dans le groupe constitué par la phosphatidylcholine, la phosphatidyléthanolamine et la phosphatidylsérine, la lysophosphatidylcholine, et leurs mélanges.

5. Formulation selon la revendication 4, dans laquelle le phospholipide est une lécithine, de préférence la lécithine de soja.

6. Formulation selon la revendication 5, dans laquelle la lécithine est présente à raison de 0,5 à 4,0 % (en poids/poids), de préférence de 1,0 % à 1,5 %, et est présente en particulier à raison d'environ 1,2 %.

7. Formulation selon la revendication 1, dans laquelle l'additif lipophile est choisi dans le groupe constitué par le cholestérol, l'acide phosphatidique et l'estéarylamine, le cholestérol étant l'additif préféré.

8. Formulation selon la revendication 7, dans laquelle la quantité d'additif lipophile est comprise entre 0,1 et 0,6 % (en poids/poids), de préférence entre 0,2 et 0,4 %, et est en particulier d'approximativement 0,3 %.

9. Formulation selon la revendication 1, dans laquelle la relation entre la quantité d'agent lipophile conservateur et la quantité de phospholipide varie de 1:1 à 1:3, et est en particulier d'approximativement 1:2,5.

10. Formulation selon la revendication 1, dans laquelle l'agent antioxydant utilisé dans la préparation des liposomes est l'α-tocophérol ou l'hydroxytoluène butylé (BHT), le BHT étant préféré à raison de 0,003 à 0,007 % (en poids/poids).

11. Formulation selon la revendication 1, dans laquelle l'agent antioxydant utilisé pour la conservation dans la forme finale est l'acide éthylènediaminetétraacétique (EDTA), l'édétate disodique et le métabisulfite de sodium, l'EDTA et le métabisulfite de sodium étant préférés à raison de 0,01 % et 0,2 %, respectivement.

12. Formulation selon la revendication 11, dans laquelle le solvant organique est un glycol, et il s'agit de préférence de propylèneglycol.

13. Formulation selon la revendication 12, dans laquelle le solvant organique est présent à raison de 8 à 15 % (en poids/poids), en particulier de 10,0 à 12,5 %, et de préférence à raison de 11,8 %.

14. Formulation selon la revendication 1, dans laquelle l'agent tampon pour maintenir le pH du composant aqueux entre 5 et 7,5 est constitué par le tris-(hydroxyméthyl)-aminométhane à raison de 0,1 % (en poids/poids) et l'acide chlorhydrique à raison de 0,02 % (en poids/poids).

15. Formulation selon la revendication 1, dans laquelle les substances conservatrices sont le méthylparabène à 0,2 % (en poids/poids) et le propylparabène à 0,02 % (en poids/poids).

16. Formulation selon la revendication 1, dans laquelle l'agent gélifiant est choisi dans le groupe constitué par l'hydroxypropylméthyl-cellulose, l'hydroxypropylcellulose, leurs mélanges ou le Carbopol 940, le Carbopol 940 étant préféré à raison de 0,85 %.

17. Formulation selon l'une quelconque des revendications 1 à 16, sous la forme finale d'un gel.
